# EUROPEAN PATENT SPECIFICATION

(11) **EP 1 382 318 B1**
(45) Date of publication and mention of the grant of the patent: **10.05.2006**
(21) Application number: 03022211.1
(22) Date of filing: 12.02.1999
(51) Int. Cl.: A61F 5/455

(54) **A urine discharge apparatus for female**
Vorrichtung zum Auffangen von Urin für Frauen
Appareil d'évacuation d'urine pour les femmes

(30) Priority: 13.02.1998 KR 4280098; 27.01.1999 KR 2595000099
(43) Date of publication of application: 21.01.2004
(62) Divisional of application: 99905343.2
(73) Proprietor: Kim, Kyoung-Hun, Pusan 600-022 (KR)
(72) Inventor: Kim, Kyoung-Hun, Pusan 600-022 (KR)
(74) Representative: TER MEER - STEINMEISTER & PARTNER GbR

(56) References cited:
- DE-A- 3 042 451
- GB-A- 2 091 560
- US-A- 4 631 061
- US-A- 4 747 166
- US-A- 5 678 564

## Description

### TECHNICAL FIELD

The present invention relates to a urine discharge apparatus for female, and particularly to the urine discharge apparatus providing the advantage that urine collected in a disposable pack of collecting member is discharged without detention by use of a small pump and it is convenient to carry and keep due to its small volume in being folded.

Generally, urination of female is different from that of male and has a problem that female should expose excessively her body for maintaining a sitting position with her trouser taken off. In order to hide the exposed body, female usually use a toilet seats in a closed space. However, in a case that a public toilet is not available at outdoor, urination of female is inevitably accompanied by an excessive exposure of body which imposes a burden on her and may evoke a gender complex.

### BACKGROUND ART

In order to minimize the excessive exposure of body and overcome the complex, a female urine discharge device is disclosed by Publication of Laid-open Korea Patent Application 95-5332. The proposed device has a funnel-shaped hemisphere and protects leakage of urine by contact portion of the hemisphere whose rim was made of soft material, a saw-toothed anti-back current means in interior surface for preventing back current of urine after urination and an outlet pipe that is wrinkled.

While not used, this device is kept in a pocket placed at underwear in a manner that the outlet pipe is attached to underwear and folded upwardly.

Since the contact portion is easily separated from the hemisphere when the device is taken out from the pocket or used, however, the device cause much inconvenience and the underwear may be spoiled by spreading of urine because urethra of female is placed at lower portion of body and thus urine is not collected in the outlet pipe protruding forward. Accordingly, a repeated use of the device is impossible and cumbersomeness that female always wear during her activity is accompanied.

In addition, since female usually urinate 7 or 8 times and deject 1 or 2 times a day, female use a toilet seat more frequently than male and thus public toilet may be overcrowded. Accordingly infection through toilet seat may take place more frequently.

The GB 2091560 A discloses female underpants with urination facility. Female underpants are formed from a material which is watertight and adherent to the body of the wearer. A crotch of the underpants is provided with a cavity facing the urinating organ of the female body and has a urine discharge port projecting from the lower end of the cavity. To this urine discharge port is detachably connected a urine discharge device provided with a pump so that the urine voided into the cavity can be discharged by forced suction produced by the urine discharge device, thereby enabling a female driver or passenger in a car to urinate with the underpants on when no lavatory is conveniently available.

### DISCLOSURE OF INVENTION

The present invention is proposed to solve the above-mentioned problem, and the object of the invention is to provide "urine discharge apparatus for female" that female carry the apparatus at normal time and attach the apparatus on perineum as pose of male urinating in the time of urination, the apparatus is also convenient to carry and keep and makes it possible to examine the amount of urine and so on and therefore has a medical effect with early detection of disease. After urination, the urine collected in the collecting member is discharged outside without detention by pump. The present invention provides a urine discharge apparatus for female having a collecting member and a body including a pump and a battery, wherein the pump includes an inlet and an outlet; wherein the body is a hollow body having a switch mounted to the body for controlling a flow of current supplied to the pump; an introduction hose is connected to the inlet and positioned outside the body; the collecting member is connected to a rear wall of the body by a hinge and having a shape of a bucket s o as to control easily the direction of urine discharged.

Another object of the present invention is to provide a urine discharge apparatus for female that use a disposable pack for sanitary urination, the disposable pack is also used for medical examination and pregnancy diagnosis. Also, another object of the present invention is to provide a urine discharge apparatus for female that enable a bedridden patient to urinate by oneself or with help of nurse in the bed.

### BRIEF DESCRIPTION OF DRAWING

Fig. 1 is an exploded perspective view showing a condition of the separated urine discharge apparatus.

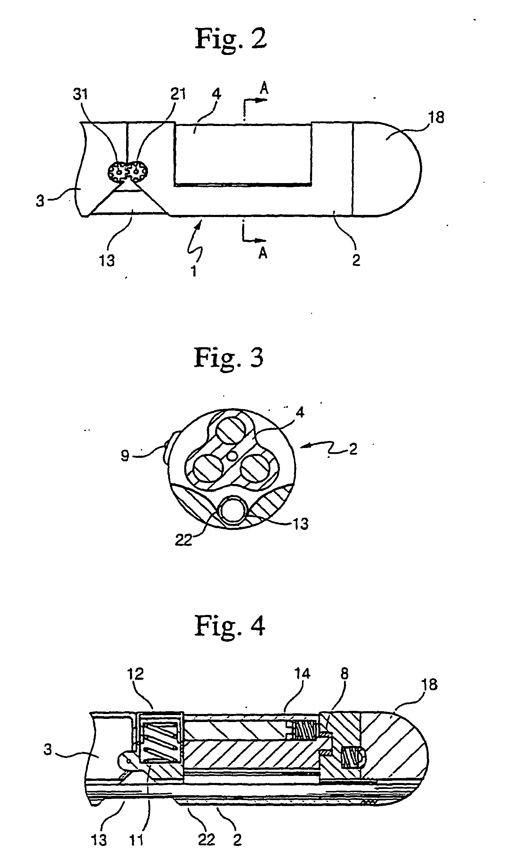

Fig. 2 is a side view showing a part of urine discharge.
Fig. 3 is a cross-sectional view taken from line A-A of the Fig. 2,
Fig. 4 is a longitudinal sectional view of the Fig. 2,
Fig. 5 is a side view showing an operational condition of urine discharge apparatus.
Fig. 6 is a side view showing a using condition of the urine discharge apparatus.
Fig. 7 is a side view showing a condition that an auxiliary hose is connected to top end of the discharge hose.
Fig. 8 is a modification of one embodiment, a partly sectional side view showing a condition that additional float-type switch for applying electric power is provided,
Fig. 9 is an exploded perspective view showing a condition of the separated urine discharge apparatus according to the present invention,
Fig. 10 and Fig. 11 are views showing an operational condition of the urine discharge apparatus of the Fig. 9,
Fig. 12 is a perspective view showing a using condition of the urine discharge apparatus of the Fig. 9,
Fig. 13 is a side view of the urine discharge apparatus of the Fig. 9, and
Fig. 14 is a bottom view of the urine discharge apparatus of the Fig. 9

The description of primary reference number is followings;

| | |
|---|---|
| 1: body | 2: driving member |
| 3: pump member | 4: receptacle |
| 5: pump | 6: collecting member |
| 7: disposable pack | 9: switch |
| 12: door | 13: discharge hose |
| 13': auxiliary hose | 15; discharge pipe |
| 16: test paper | 41, 42: receptacle |
| 51: impeller | 52: entrance |
| 53: outlet | 62: magnet |
| 81: groove | 82: rear wall |
| 83: clip | 831: blade portion |
| 85: bottom | 87: display |
| 88. holder | 131: introduce hose |

### BEST MODE FOR CARRING OUT THE INVENTION

According to the present invention, an urine discharge apparatus for female in accordance with claim 1 is disclosed. According to a preferred embodiment, the pump includes a cylindrical casing; an electromagnet is formed by winding a coil around the casing; a cylindrical sleeve is mounted in the casing; and a magnet is mounted in an outer circumference of the sleeve; and a rotating blade mounted in the sleeve. Further the pump includes a shaft mounted on an axis of the rotating blade and a guide disk is mounted to the shaft, whereas a plurality of flaps protrudes from an outer circumference of the guide disk. Further the pump includes an auxiliary centrifugal impeller which is mounted to the shaft.
The urine discharge apparatus for female according to the present invention comprises the body having a hole at the rear wall thereof and the pump is removable combined to the hole.
Moreover a urine discharge apparatus for female according to the present invention may comprise a hook, which is formed at the inlet of the body and a groove is formed inside of the hole for hooking and releasing the hook.
Further a urine discharge apparatus for female includes a clip, which is mounted to a lower surface of the body.
The urine discharge apparatus for female further comprises the body including a system with a flow measuring instrument for measuring the flow of urine, a battery indicator for indicating a remaining life of a battery connected to the switch and a timepiece; and wherein a display is installed in basement of the body for representing contents from the system as a text or figure.
In a further embodiment of the present invention the urine discharge apparatus for female includes a disposable pack, which is mounted in the collecting member, wherein the disposable pack could further comprise a non-woven fabric, which is attached within the disposable pack.
A urine discharge apparatus for female according to an embodiment of the present invention includes a flange extending longitudinally along an edge of a side wall of the collecting member and bending inwardly is integrated with the disposable pack.
Moreover a urine discharge apparatus for female according an embodiment of the present invention comprises a metallic hose clip, which is mounted to the introduce hose.
Further a urine discharge apparatus for female comprising a magnet, which is mounted inside of the collecting member for removable mounting the hose clip thereto.

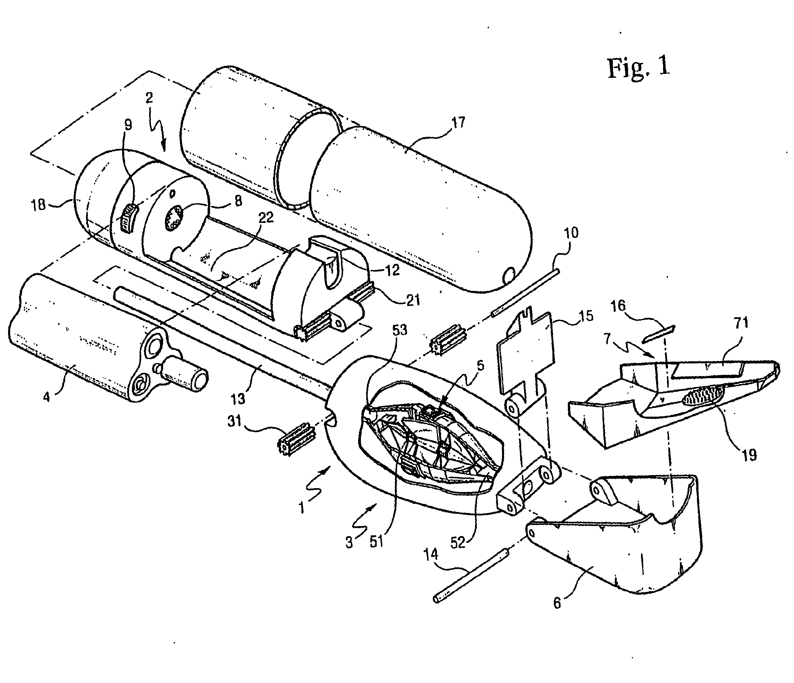

Fig. 1 is an exploded perspective view showing a condition of separated urine discharge apparatus for female, a body 1 is divided into the driving member 2 and the pump member 3, and these divided member are connected to corresponding portion by hinge means of pin 10 to cooperate each other in manner that gear 21 engages with gear 31.

The driving member 2 of the body 1 has a space for carrying the battery case 4, and the holding clip 8 is provided in the centre of both sides thereof so as to restrict rotation of the receptacle 4.

And, the switch 9 for switching electric power is provided on outer surface of the driving member 2 and may be located on right or left side depending on user in order that user can operate the switch 9 with thumb, a door 12 which opens and closes the entrance of the battery case 4 by a spring 11, which is provided on opposing side of the switch 9, a through passage 22 is formed in the bottom of the driving member 2, a discharge hose 13 is inserted into through passage and protrude outward from end of the driving member 2.

The pump member 3 of the body 1 have a space for containing a small pump 5 and its one side is connected to the collecting member 6 by hinge means of a pin 14 to cooperate with the collecting member 6, the pump 5 has the discharge pipe 15 and the discharge hose 13 at both side.

The collecting member 6 is connected to pump member 3 by hinge means of pin 14 and upwardly folded in the time of keeping and carrying, and may be unfolded to a predetermined angle when used.

The discharge pipe 15 provided in the middle of hinge means connects the pump member 3 to the collecting member 6 and can rotate independently, a flange at both sides of the discharge pipe 15 contacts completely with the surface of the collecting member 6 to support it, and the discharge pipe 15 provides a passage for introducing the urine collected in the collecting member 6 to inlet 52 of the pump 5.

The pump 5 is incorporated in the pump member 3 of the body 1 and is provided with impeller 51 at inside thereof, the impeller 51 rotates by electric power supplied through the switch 9 to discharge urine, and the pump 5 has the inlet 52 communicating with the discharge pipe 15 in one side and the outlet 53 communicating with the discharge hose 13 in the other side. The pump 5 above mentioned is not limited to a typical pump generally used for urine discharge, gear pump and so on, may be used also. The battery case 4 contains three 1.5 V batteries, and is fitted in the holding clip 8 for restricting the battery case 4, a positive pole of the battery is connected to the switch 9 and a negative pole to the pump 5 in normal way.

Also, a rechargeable cell and charger may be used in stead of the battery. The switch 9 operates the pump 5 by switching electric power, and may be replaced by an adjustable resistance switch if control of rotation of the pump 5 is necessary.

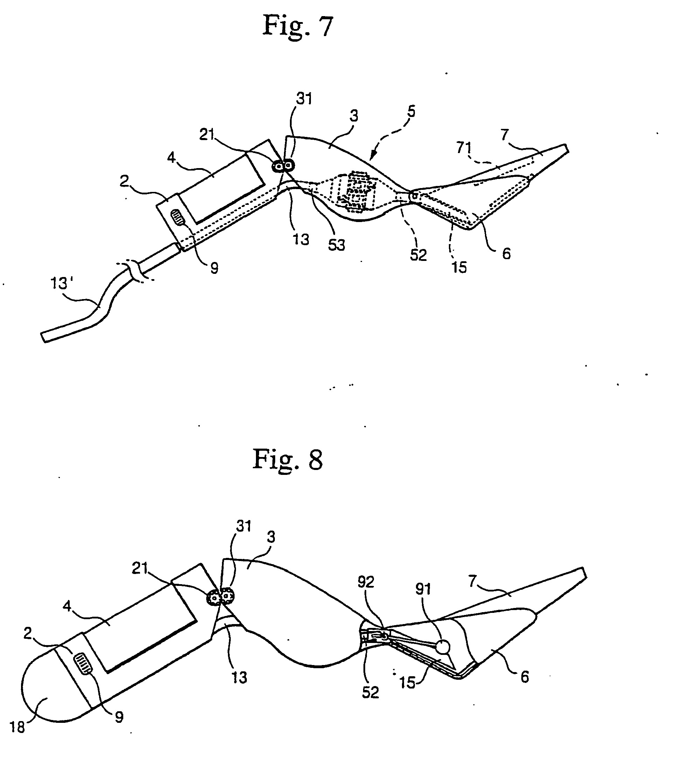

Also, as shown in Fig.8, a floating- type switch 92 may be additionally provided so as to connect parallel a floater 91 and the switch 9 of the driving member 2, the floater 91 is raised from a surface of the collecting member as soon as an urine is collected in the collecting member 6.

By above movement, the float-type switch 92 may be operate and supply automatically electric power to drive the pump 5.

This operation makes it possible to discharge urine independently of the switch 9 of the driving member 2.

The disposable pack 7 mounted on the collecting member 6 is sold and kept in folded condition. When used, the disposable pack 7 is unfolded to obtain space for collecting urine and is attached to perineum. The flange is provided longitudinally at both side walls so as to prevent urine discharged from urethra from spreading and collect urine easily.

Also, non-woven fabric may be attached on bottom of the disposable pack 7 to obtain the effect of absorption and prevent spread of urine. Further, test paper 16 may be attached inside of the disposable pack 7 in order that user, if necessary, can examine a proteinuira, a haematouria, a glycosuria and a pregnancy for private checkup.

On the other hand, the cover 17 made of resin or fabric may be used to wrap the driving member 2 of the body 1 and pump member 3 for protection and aesthetic pleasure. As shown in Fig.7, the auxiliary hose 13' may be connected to top end of the discharge hose 13 protruding outward to ensure a bedridden patient can urinate in bed with help of nurse or by oneself, and thus the discharged urine may be easily collected in other drainage or urinal. The reference number 18 in drawings is a cap connected to the top end of driving member 2 of the body 1.

The using method of the above mentioned urine discharge apparatus will be described in followings.

The battery case 4 receives a battery while rotate repeatedly about a third of one cycle after entrance of the receptacle 4 is opened by lowering the door 12 of the driving member 2, and the driving member 2, the pump member 3 and the collecting member 6 is adjusted to the same angle that the male urinate.

The collecting member 6 is attached to perineum after the discharge pipe 15 contacts the surface of the collecting member 6, and the pump 5 will be rotate simultaneously with urination or right before urination while the driving member 2 and the pump member 3 is adapted to convenient angle for urination.

The urine discharged from urethra is introduced to the discharge pipe by sucking operation of the pump 5 as soon as collected in the collecting member 6, and continuously flow from the inlet 52 of the pump 5 and impeller 51 to the outlet 53, and discharged outward by eject operation of the pump 5 through discharge hose 13.

After urine collected in the collecting member 6 is completely discharged, the operation of the pump 5 stops by switching off switch 9. Thus an operating cycle of the urine discharge apparatus is finished and the apparatus may be folded for carrying or keeping.

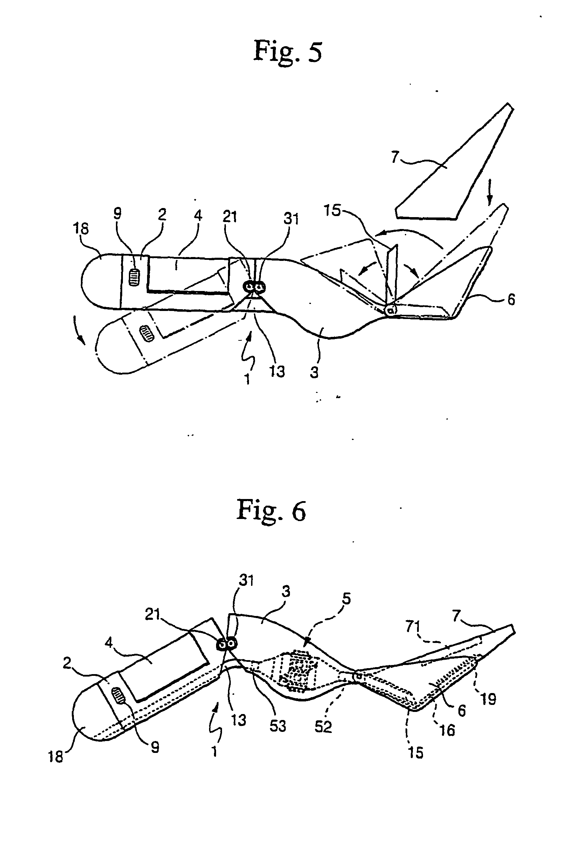

Also, in case the disposable pack 7 is used as shown in the Fig. 5 and 6, a using method of the apparatus is the same with that of the above-mentioned case excepting the disposable pack 7 is compressed by the discharge pipe 15.

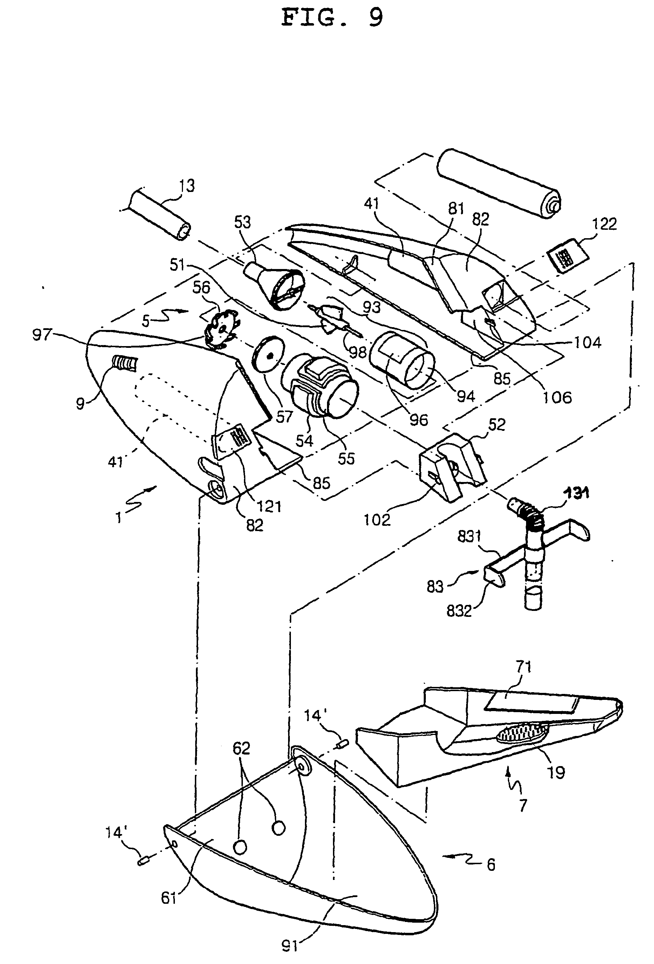

Further, the use of the disposable pack 7 may ensure more sanitary urination because the urine is absorbed in the non-woven fabric 19 without spreading and the flange 71 extending longitudinally in side wall also prevents effectively spreading of urine. Further test paper 16 attached inside of the disposable pack 7 enables a user to check easily health index. On the other hand, when a bedridden patient urinates in bed with help of nurse or for oneself, as shown in Fig. 9, the auxiliary hose 13' may be connected to top end of the discharge hose 13 protruding outward from the through passage 22 of the driving member 2 after a cap coupled with the top end of the driving member 2 is removed, thus the discharged urine can be easily collected in other drainage or urinal.

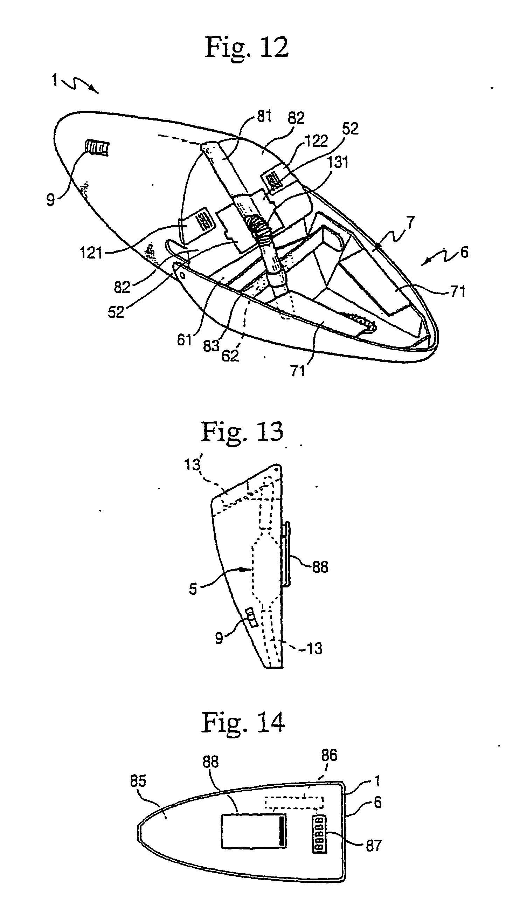

Fig. 9 to Fig. 14 represent an urine discharge apparatus according to the present invention, the apparatus has a structure that the pump member 3 and the driving member 2 for operating the pump member 3 are incorporated into the body 1 to be unitary shape.

In this embodiment, a collecting member 6 has a form of hemi-cone with spherical basement and is connected to both sides of the rear wall 82 by a hinge; a body 1 has the same shape as the collecting member 6 so that the body 1 rotates to be received into the collecting member 6; a pump 5 having an inlet 52 and an outlet 53 is arranged longitudinally to an introduce hose 131 connected to the inlet 52, which extends to inferior of the collecting member 6; a discharge hose 13 connected to outlet 53 of the pump 5 is arranged so as to discharge the urine outward; a groove 81 is formed at a central line of the rear wall 82 of the body 1 so as to contain the introduce hose 131; a switch 9 supplying the pump 5 with electric power is mounted on the outside of the body 1; a receptacle 41, 42 having a door is formed at both interior of the rear wall 82 so that the receptacle 41, 42 receives a battery; a hose clip 83 having a blade portion 831 at both sides is combined with the introduce hose 131; a magnet 62 is attached to a front wall 61 of the collecting member 6 so that the clip may be clung completely to the magnet 62.

The hose clip 83 has a guide portion 832 folded about 90 degrees at both sides of the blade portion 831 so that the disposable pack 7 may be unfolded completely in accordance with a shape of the collecting member 6, and thus the collecting member 6 including the disposable pack 7 can collect the urine entirely.

A display 87 represents the contents of a battery indicator and a flow measuring instrument, installed optionally inside of the body 1, as a text or figure is provided in the basement the body 1 and clip 88 is also provided for carrying.

Fig. 9 shows the urine discharge apparatus according to the present invention. It shows a condition of the separated apparatus in an exploded perspective view. The apparatus comprises the pump 5 for discharging the urine, the switch 9 for operating the pump 5, the body 1 contains a battery and the pump 5, wherein the collecting member 6 is connected to the body 1 by a hinge.

The collecting member 6 has a conical form, which is cut longitudinally with its cross section turned up, and the basement 85 of the collecting member 6 is in the shape of sphere, thus this member is easily attached to perineum.

Both sides of the front wall 61 of the collecting member 6 are connected to the outside of the rear wall 82 by pin 14', and thus the collecting member 6 is folded in being carried or kept and unfolded to predetermined angle in the time of use.

The disposable pack 7 inserted into the collecting member 6 is kept and sold with folded condition and is unfolded in the time of use so that this pack may gain the space for collecting urine discharged from urethra. Also the disposable pack 7 has a flange 71 extending longitudinally along the edge of the side wall so as to protect the urine from spreading, whereas a non-woven fabric 19 is attached to the bottom of the disposable pack 7.

The body 1 has the same shape as the collecting member 6 with the base having a curved surface. Although the body 1 in Fig. 1 has the structure that is split longitudinally along the center line into two portions, this structure is not limitative and may be form that the basement 85 is split laterally.

Moreover, the body 1 is formed to be inserted into the collecting member 6 so as to minimize its volume when the urine discharge apparatus is kept or carried, and the pump 5 which contains impeller 51 and has the inlet 52 and outlet 53 at both sides is arranged longitudinally at a center line thereof.

The inlet 52 of the pump 5 extends to the outside of the rear wall 82 and can be attachable and detachable, the introduce hose 131 is connected to this inlet 52 and the discharge hose 13 to the outlet 53 and the end of the introduce hose 131 is fitted tightly in the inlet 52 of the pump 5 so as to prevent a liquid from permeating, whereas the other end extends to the inside of the collecting member 6.

The introduce hose 131 made of a synthetic resin has a wrinkle at its central part so that the introduce hose 131 is flexible.

The discharge hose 13 is connected to a front end of the body 1 for discharging urine outward and may be provided with an auxiliary hose for facilitating bedridden patients to urinate in bed with help of nurse or for oneself. To this end, the discharge hose 13 is preferably made of hard synthetic resin.

The groove 81 for containing the introduce hose 131, when the collecting member 6 is folded is formed at the rear wall 82 of the body 1 so that the urine discharge apparatus can minimize its volume in being folded. The switch 9 for supplying the pump 5 with electricity power is mounted on one side of the body 1 in accord with a user and the receptacles 41, 42 having doors 121, 122 are arranged at both inner sides of the body 1.

On the other hand, the metallic clip 83 having the blade portion 831 at both sides is combined with the introduce hose 131 extending to introduce the urine collected in the collecting member 6, the hose clip 83 helps the end of the introducing hose 131 turn toward to bottom of the collecting member 6 while holding the disposable pack 7, and thus urine collected in the collecting member 6 is introduced without detention.

Moreover the guide portion 832 is provided as one body so as to assure that the disposable pack 7 maintains the condition of being completely unfolded, and an auxiliary steel plate or wire may be combined. It is possible to modify the shape of the guide portion 832 if this modification promotes the restriction of the guide portion 832.

Furthermore, the magnet 62 is provided on the front wall 61 of the collecting member 6 to fix the metallic hose clip 83 combined with the introduce hose 131, and both the hose clip 83 attached to the front wall 61 and the introduce hose 131 inserted in the hose clip 83 follow the movement of the collecting member 6 during the rotation of the collecting member 6.

The pump 5 installed in the body 1, as one embodiment, comprises a casing 55 provided with a electromagnet 54 wound by coil around it, a cylindrical impeller 51 is arranged inside of the casing 55, wherein a screw assembly is installed inside of the cylindrical impeller 51, a guide disc 56 having a plurality of flaps 97 on an outer circumference thereof for guiding a flow of urine and a centrifugal auxiliary impeller 57.

When the coil arranged at outer surface of the electromagnet 54 is supplied with electricity power through the switch 9, one polarity occurs simultaneously at the electromagnet 54. Then the impeller 51 is repelled by the same polarity as the electromagnet 54 so that the impeller 51 rotates and discharges the urine. The construction of the pump 5 is not limitative, and a typical pump may be used if it can discharge the urine.

The impeller 51 includes a cylinder sleeve 94 having a diameter less than the casing 55. A magnet with N-pole and S-pole is filled in the circumferential surface of the casing 55, whereby the magnet with N pole and S pole is filled in an outer circumference of the sleeve 94, a rotating blade 93 is arranged in the sleeve 94 and a shaft 98 is combined to an axis of the rotating blade 93 to be a rotating centre.

And the pump 5 is preferably removable from the body 1 with the introduce hose 131 and discharge hose 13 so that it is possible to disinfect and replace pump 5.

Therefore, in order to separate the pump 5, a hole 106 is formed through the rear wall 82 and a groove 104 is formed at an inner wall of the hole 106. At both side walls of the inlet 52, hooks 102 are formed to be inserted into the grooves 104, respectively.

Also typical system like a flow measuring instrument, a battery indicator and a timepiece may by optionally incorporated in the body 1, and a display 87 which represent contents from the system as a text or figure may be installed in the basement 85 of the body 1.

These systems or display 87 supply the data necessary to remedy. The clip 88 is also provided on the basement 85 for carrying.

The using method of the above mentioned urine discharge apparatus will be described in followings.

A battery is inserted into the receptacle 41, 42 after the door 121, 122 arranged at the rear wall 82 is open and then the collecting member 6 is adjusted to some angle that is convenient to urinate. Then the pump 5 rotates simultaneously with urination or right before urination by switching on switch 9.

The urine discharged from urethra is introduced to the introduce hose 131 by sucking operation of the pump 5 as soon as collected in the collecting member 6, and continuously flow to the outlet 53 through the inlet 52 and impeller 51, and discharged outward by eject of the pump 5 through discharge hose 13.

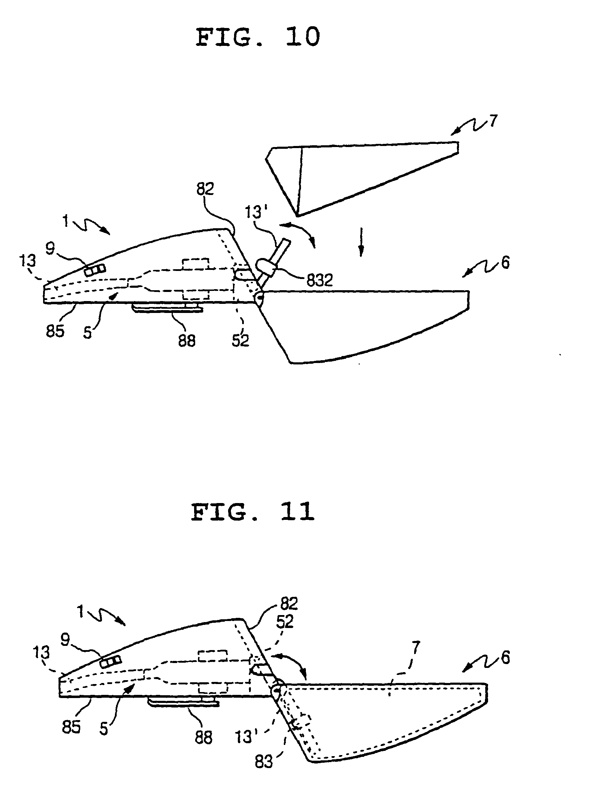

After the urine collected in the collecting member 6 is completely discharged the operation of the pump 5 stops by switching off the switch 9. Thus an operation cycle of the urine discharge apparatus is finished and the apparatus may be folded to be carried and kept. When the disposable pack 7 is used as Fig. 10 and Fig. 11 the disposable pack 7 is tightly attached to the collecting member 6 by a magnetic force of the magnet 62.

In this case, the urine can be absorbed in the non-woven fabric 19 without spreading. The flange 71 arranged at both sides in the rim of the disposable pack 7 prevents more effectively the urine from spreading. The urine beyond the absorbability of the non-woven fabric 19 flows down to be collected in the lower portion of the disposable pack 7.

This discharge process with the disposable pack 7 is more sanitary than that without the disposable pack 7.

Also as the system 86 provides information including the amount of the urine, time to charge the battery and time, and these information is represented on the display 87 arranged in the basement 85 of the body 1 as a text or figure, it is possible to cure effectively and to manage and wear the apparatus with ease.

As above mentioned, the apparatus according to the present invention is not only simple in construction but also adjusted easily to a convenient angle to use, and can protect the body from being infected by toilet seats by use of the disposable pack.

Moreover, the disabled patient is able to urinate easily by this apparatus, and the urine discharge apparatus provides the advantage that a user can examine by oneself the health index and pregnancy for health care.

Moreover the apparatus is convenient to carry and keep due to its small volume in being folded and provide advantages that a user can detect a disease in early stages by the analysis of the urine, and thus enhance a medical efficiency.

## Claims

1. A urine discharge apparatus for a female comprising:
a collection member (6) and a body (1) including a pump (5) and a battery, wherein the pump (5) includes an inlet (52) and an outlet (53);
**characterized in that** the collection member (6) has the form of a cone which is cut longitudinally with
its cross section turned up and a base of the collection member (6) has the form of a sphere with a substantially triangle form;
the body (1) has the same form as the collection member (6);
a switch (9) is mounted to the body (1) for controlling a flow of current supplied to the pump (5); an introduction hose (131) is connected to the collection member (6) and to the inlet (52); and the collection member (6) is connected to a rear wall (82) of the body (1) by a hinge, wherein when rotating body (1) and collection member (6) by the hinge the body (1) is covered by the collection member (6).

2. The urine discharge apparatus as claimed in claim 1, wherein the pump (5) includes a cylindrical casing (55); an electromagnet (54) is formed by winding a coil around the casing (55); a cylindrical sleeve (94) is mounted in the casing (55); a magnet (96) is mounted in an outer circumference of the sleeve (94; and a rotating blade (93) is mounted in the sleeve (94).

3. The urine discharge apparatus as claimed in claim 2, wherein the pump (5) includes a shaft (98) mounted on an axis of the rotating blade (93) and a guide disk (56) is mounted to the shaft (98).

4. The urine discharge apparatus as claimed in claim 3, wherein a plurality of flaps (97) protruding from an outer circumference of the guide disk (56).

5. The urine discharge apparatus as claimed in claim 3, wherein the pump (5) includes an auxiliary centrifugal impeller (57) mounted to the shaft (98).

6. A urine discharge apparatus for female as claimed in claim 1, wherein the body (1) has a hole (106) at the rear wall (82) thereof and the pump (5) is removable combined to the hole (106).

7. A urine discharge apparatus for female as claimed in claim 6, wherein a hook (102) is formed at the inlet (52) of the body (1) and a groove (104) is formed in inside of the hole (106) for hooking and releasing the hook (102).

8. A urine discharge apparatus for female as claimed in claim 1, wherein a clip (88) is mounted to a lower surface (85) of the body (1).

9. A urine discharge apparatus for female as claimed in one of the claims 1, 2, 3, or 4, wherein the body (1) includes a system (86) including a flow measuring instrument for measuring the flow of urine, a battery indicator for indicating a remaining life of a battery connected to the switch (9) and a timepiece; and wherein a display (87) is installed in basement (85) of the body (1) for representing contents from the system (86) as a text or figure.

10. A urine discharge apparatus for female as claimed in claim 1, wherein a disposable pack (7) is mounted in the collecting member (6).

11. A urine discharge apparatus for female as claimed in claim 10, wherein a non-woven fabric (19) is attached within the disposable pack (7).

12. A urine discharge apparatus for female as claimed in claim 11, wherein a flange (71) extending longitudinally along an edge of a side wall of the collecting member (6) and bending inwardly is integrated with the disposable pack (7).

13. A urine discharge apparatus for female as claimed in one of the claims 1, 2, 3 or 4, wherein a metallic hose clip (83) is mounted to the introduce hose (131).

14. A urine discharge apparatus for female as claimed in claim 13, wherein a magnet (62) is mounted inside of the collecting member (6) for removable mounting the hose clip (83) thereto.

## Patentansprüche

1. Urinentleerungsvorrichtung für Frauen, mit:
einem Sammelelement (6) und einem Körper (1), der eine Pumpe (5) und eine Batterie enthält, wobei die Pumpe (5) einen Einlass (52) und einen Auslass (53) aufweist;
**dadurch gekennzeichnet, dass**
das Sammelelement (6) die Form eines Kegels hat, der in Längsrichtung geschnitten ist und dessen Schnittfläche nach oben gewendet ist, wobei die Basis des Sammelelements (6) die Form einer Kugel mit einer im Wesentlichen dreieckigen Form hat;
der Körper (1) die gleiche Form wie das Sammelelement (6) hat;
am Körper (1) ein Schalter (9) angebracht ist, um einen Stromfluss zu der Pumpe (5) zu steuern; ein Einführungsschlauch (131) mit dem Sammelelement (6) und mit dem Einlass (52) verbunden ist; und das Sammelelement (6) durch ein Scharnier mit einer Rückwand (82) des Körpers (1) verbunden ist, wobei der Körper (1) dann, wenn er in Bezug auf das Sammelelement (6) durch das Scharnier gedreht wird, von dem Sammelelement (6) abgedeckt ist.

2. Urinentleerungsvorrichtung nach Anspruch 1, bei der die Pumpe (5) ein zylindrisches Gehäuse (55) aufweist; ein Elektromagnet (54) durch Wickeln einer Spule um das Gehäuse (55) gebildet ist; in dem Gehäuse (55) eine zylindrische Hülse (94) angebracht ist; in einem äußeren Umfang der Hülse (94) ein Magnet (96) angebracht ist; und in der Hülse (94) ein drehbares Schaufelrad (93) angebracht ist.

3. Urinentleerungsvorrichtung nach Anspruch 2, bei der die Pumpe (5) eine an der Achse des drehbaren Schaufelrades (53) angebrachte Welle (98) und eine an der Welle (98) angebrachte Leitscheibe (56) aufweist.

4. Urinentleerungsvorrichtung nach Anspruch 3, bei der vom äußeren Umfang der Leitscheibe (56) mehrere Klappen (97) vorstehen.

5. Urinentleerungsvorrichtung nach Anspruch 3, bei der die Pumpe (5) ein zusätzliches Zentrifugalförderelement (57) aufweist, das an der Welle (98) angebracht ist.

6. Urinentleerungsvorrichtung für Frauen nach Anspruch 1, bei der der Körper (1) in seiner Rückwand (82) ein Loch (106) aufweist und die Pumpe (5) mit dem Loch (106) lösbar kombiniert ist.

7. Urinentleerungsvorrichtung für Frauen nach Anspruch 6, bei der am Einlass (52) des Körpers (1) ein Haken (102) ausgebildet ist und in dem Loch (106) eine Nut (104) ausgebildet ist, um den Haken (102) einzuhaken und freizugeben.

8. Urinentleerungsvorrichtung für Frauen nach Anspruch 1, bei der an einer unteren Oberfläche (85) des Körpers (1) eine Lasche (88) angebracht ist.

9. Urinentleerungsvorrichtung für Frauen nach einem der Ansprüche 1, 2, 3 oder 4, bei der der Körper (1) ein System (86) umfasst, das ein Durchflussmessinstrument zum Messen des Urindurchflusses, einen Batterieanzeiger, der die verbleibende Lebensdauer einer mit dem Schalter (9) verbundenen Batterie anzeigt, und eine Uhr enthält; und bei der eine Anzeige (87) im Unterbau (85) des Körpers (1) installiert ist, die die Inhalte von dem System (86) als Text oder als Figur darstellt.

10. Urinentleerungsvorrichtung für Frauen nach Anspruch 1, bei der in dem Sammelelement (6) ein Wegwerfbeutel (7) angebracht ist.

11. Urinentleerungsvorrichtung für Frauen nach Anspruch 10, bei der in dem Wegwerfbeutel (7) ein Vliesstoff (19) angebracht ist.

12. Urinentleerungsvorrichtung für Frauen nach Anspruch 11, bei der in den Wegwerfbeutel (7) ein Flansch (71) integriert ist, der sich in Längsrichtung längs einer Kante einer Seitenwand des Sammelelements (6) erstreckt und nach innen gebogen ist.

13. Urinentleerungsvorrichtung für Frauen nach einem der Ansprüche 1, 2, 3 oder 4, bei der an dem Einführungsschlauch (131) eine metallische Schlauchklemme (83) angebracht ist.

14. Urinentleerungsvorrichtung für Frauen nach Anspruch 13, bei der in dem Sammelelement (6) ein Magnet (82) angebracht ist, um die Schlauchklemme (83) lösbar daran anzubringen.

## Revendications

1. Appareil d'évacuation d'urine destiné à une femme, comprenant :
un élément de collecte (6) et un corps (1) comprenant une pompe (5) et une pile, dans lequel la pompe (5) comprend une entrée (52) et une sortie (53) ;
**caractérisé en ce que** :
- l'élément de collecte (6) a la forme d'un cône qui est coupé longitudinalement avec sa section transversale retournée et une base de l'élément de collecte (6) a la forme d'une sphère avec une forme sensiblement triangulaire ;
- le corps (1) a la même forme que l'élément de collecte (6) ;
- un interrupteur (9) est monté sur le corps (1) pour commander un écoulement de courant alimenté à la pompe (5) ; un flexible d'introduction (131) est raccordé à l'élément de collecte (6) et à l'entrée (52) ; et l'élément de collecte (6) est raccordé à une paroi arrière (82) du corps (1) par une articulation, dans lequel lorsque l'on fait tourner le corps (1) et l'élément de collecte (6) avec l'articulation, le corps (1) est recouvert par l'élément de collecte (6).

2. Appareil d'évacuation d'urine selon la revendication 1, dans lequel la pompe (5) comprend un boîtier cylindrique (55) ; un électroaimant (54) est formé en enroulant une bobine autour du boîtier (55) ; un manchon cylindrique (94) est monté dans le boîtier (55) ; un aimant (96) est monté dans une circonférence externe du manchon (94) ; et une pale rotative (93) est montée dans le manchon (94).

3. Appareil d'évacuation d'urine selon la revendication 2, dans lequel la pompe (5) comprend un arbre (98) monté sur un axe de la pale rotative (93) et un disque de guidage (56) est monté sur l'arbre (98).

4. Appareil d'évacuation d'urine selon la revendication 3, dans lequel une pluralité de rabats (97) fait saillie à partir d'une circonférence externe du disque de guidage (56).

5. Appareil d'évacuation d'urine selon la revendication 3, dans lequel la pompe (5) comprend une roue centrifuge auxiliaire (57) montée sur l'arbre (98).

6. Appareil d'évacuation d'urine pour femmes selon la revendication 1, dans lequel le corps (1) a un trou (106) au niveau de sa paroi arrière (82) et la pompe (5) est combinée de manière amovible sur le trou (106).

7. Appareil d'évacuation d'urine pour femmes selon la revendication 6, dans lequel un crochet (102) est formé au niveau de l'entrée (52) du corps (1) et une rainure (104) est formée à l'intérieur du trou (106) pour accrocher et libérer le crochet (102).

8. Appareil d'évacuation d'urine pour femmes selon la revendication 1, dans lequel une attache (88) est montée sur une surface inférieure (85) du corps (1).

9. Appareil d'évacuation d'urine pour femmes selon l'une des revendications 1, 2, 3 ou 4, dans lequel le corps (1) comprend un système (86) comprenant un instrument de mesure d'écoulement pour mesurer l'écoulement de l'urine, un indicateur de batterie pour indiquer la durée de vie restante d'une pile raccordée à l'interrupteur (9) et une horloge ; et dans lequel un écran (87) est installé dans le socle (85) du corps (1) pour représenter le contenu du système (86) sous forme de texte ou de figure.

10. Appareil d'évacuation d'urine pour femmes selon la revendication 1, dans lequel une compresse jetable (7) est montée dans l'élément de collecte (6).

11. Appareil d'évacuation d'urine pour femmes selon la revendication 10, dans lequel un tissu non tissé (19) est fixé dans la compresse jetable (7).

12. Appareil d'évacuation d'urine pour femmes selon la revendication 11, dans lequel un rebord (71) s'étendant longitudinalement le long d'un bord d'une paroi latérale de l'élément de collecte (6) et se pliant vers l'intérieur est intégré avec la compresse jetable (7).

13. Appareil d'évacuation d'urine pour femmes selon l'une des revendications 1, 2, 3 ou 4, dans lequel une attache de flexible métallique (83) est montée sur le flexible d'introduction (131).

14. Appareil d'évacuation d'urine pour femmes selon la revendication 13, dans lequel un aimant (62) est monté à l'intérieur de l'élément de collecte (6) pour monter de manière amovible l'attache (83) de flexible sur celui-ci.
